# FASCICULE DE BREVET EUROPEEN

(11) **EP 2 268 403 B1**
(45) Date de publication et mention de la délivrance du brevet: **14.02.2018**
(21) Numéro de dépôt: 09731090.8
(22) Date de dépôt: 24.03.2009
(51) Int. Cl.: B01L 3/00, A61B 5/151

(54) **DISPOSITIF D'ALIQUOTAGE ET DE FILTRAGE DU SANG**
VORRICHTUNG ZUR ALIQUOTIERUNG UND FILTERUNG VON BLUT
DEVICE FOR ALIQUOTING AND FILTERING BLOOD

(30) Priorité: 31.03.2008 FR 0801776
(43) Date de publication de la demande: 05.01.2011
(73) Titulaire: Commissariat à l'Énergie Atomique et aux Énergies Alternatives, 75015 Paris (FR)
(72) Inventeur: ROSTAING, Hervé, F-38420 Le Versoud (FR)
(74) Mandataire: Gevers & Orès
(86) Numéro de dépôt international: PCT/FR2009/000321
(87) Numéro de publication internationale: WO 2009/125092

(56) Documents cités:
- EP-A- 0 269 240
- EP-A- 0 470 438
- EP-A- 1 652 551
- EP-A- 1 692 999
- WO-A-94/09366
- US-A- 4 756 884
- US-A- 5 916 521
- US-A1- 2001 039 057
- US-A1- 2007 213 638

## Description

L'invention porte sur un dispositif d'aliquotage, de filtrage et de mesure du taux d'hématocrite du sang d'un patient. Plus particulièrement, le dispositif de l'invention permet d'aliquoter, filtrer, dispenser des faibles quantités (microlitres) de sang et mesurer le taux d'hématocrite du sang d'un patient.

Des nombreuses techniques d'analyse biomédicale nécessitent la filtration d'une faible quantité de sang pour séparer le plasma à analyser des cellules sanguines (globules rouges et blanc essentiellement). Une méthode connue pour effectuer un tel filtrage consiste à remplir de sang entier un contenant (tel qu'un cône de pipette ou un capillaire) ayant un volume donné, puis dispenser le contenu sur un filtre pour effectuer la séparation et récupérer le plasma séparé des composantes solides du sang. Il faut donc deux étapes distinctes - trois si on considère également le prélèvement du sang du corps du patient.

La société KABE Labortechnik commercialise des microtubes, à l'extérieur desquels fait saillie un conduit capillaire de volume calibré (voir une brochure à l'adresse Internet : http://www.kabe-labortechnik.de/download/kapillarblut_en.pdf). Pour prélever un volume déterminé de sang (de l'ordre de 100 µl) à l'aide d'un tel dispositif et le déposer sur un filtre on procède comme suit. Premièrement, on pique le doigt d'un patient avec une lancette, et on fait sortir une gouttelette de sang ; ensuite, on approche de la gouttelette l'extrémité du conduit capillaire, maintenu en position horizontale. Le tube se remplit progressivement en fonction du débit capillaire. Une fois rempli, le microtube est amené en position verticale pour vider le conduit capillaire sur un filtre, le vidage étant assisté par des légers chocs ou des vibrations.

L'opération est relativement complexe et ne permet pas d'éviter tout risque de contamination du ou par le sang. En outre, les forces capillaires retiennent environ 25% du liquide dans le conduit, ce qui affecte la précision de l'aliquotage.

Il est également possible de remplacer le microtube par une poire de pipette, de manière à permettre le vidage du conduit capillaire par application d'une surpression. Cette solution permet un vidage plus complet et donc un aliquotage plus précis, mais les autres inconvénients du dispositif Un dispositif de l'art antérieur est divulgué dans US-A-2001/0039057. L'invention vise à résoudre au moins certains des inconvénients de l'art antérieur.

Le dispositif de l'invention permet un aliquotage précis du sang et une dispense sensiblement complète vers un filtre, qui s'effectue de manière automatique, par capillarité.

Conformément à l'invention, ces résultats sont obtenus par un dispositif d'aliquotage et de filtrage du sang selon la revendication 1. Un tel dispositif comporte un élément de collecte de sang ; un conduit capillaire présentant une force de capillarité supérieure à celle dudit élément de collecte et en communication fluidique avec lui ; et un élément de dispense présentant une force de capillarité supérieure à celle du conduit capillaire et en communication fluidique avec lui ; ledit élément de dispense comportant un dispositif de filtrage pour séparer des globules sanguins du plasma.

Des modes de réalisation particuliers de l'invention constituent l'objet des revendications dépendantes.

Dans un mode de réalisation préféré de l'invention, les fonctions d'aliquotage, dispense et filtrage, voire également de prélèvement, peuvent être intégrées dans un même dispositif planaire de structure très simple.

Plusieurs applications du dispositif de l'invention peuvent être envisagées.

Selon une première application, ledit dispositif de filtrage peut être adapté pour se saturer en globules après le filtrage d'un volume de sang dépendant du taux d'hématocrite du dit sang mais, en tout cas, inférieur à la contenance dudit élément de collecte et dudit conduit capillaire ; des repères visuels étant prévus, permettant d'estimer par simple inspection le volume de liquide présent dans ledit conduit capillaire. De cette manière, le taux d'hématocrite dudit sang peut être déterminé à partir de l'estimation du volume de sang restant dans le conduit capillaire après saturation du dispositif de filtrage.

Selon une deuxième application de l'invention, le dispositif peut comporter également, en aval dudit dispositif de filtrage, un élément détachable en matériau absorbant le plasma sanguin et apte à conserver, après séchage, les protéines contenues dans ledit plasma. De cette manière, un échantillon de plasma peut être prélevé directement par le patient, à son domicile. Après désolidarisation du dispositif et séchage, l'élément absorbant peut être envoyé par la poste à un laboratoire pour que soit effectué une analyse des protéines du plasma qui y sont conservées.

D'autres caractéristiques, détails et avantages de l'invention ressortiront à la lecture de la description faite en référence aux dessins annexés donnés à titre d'exemple et qui représentent, respectivement :
- les figures 1 et 2, respectivement, une vue en plan et en coupe de la section d'aliquotage et dispense du sang d'un dispositif selon un premier mode de réalisation de l'invention ;
- la figure 3, une vue en coupe de la section d'aliquotage et dispense du sang d'un dispositif selon un deuxième mode de réalisation de l'invention ;
- les figures 4A à 4E, le remplissage de la section d'aliquotage et dispense du sang des figures 1 et 2 ;
- les figures 4F à 4I, le vidage de cette même section ; et
- la figure 5, l'application d'un dispositif de l'invention à la mesure du taux d'hématocrite d'un échantillon de sang.

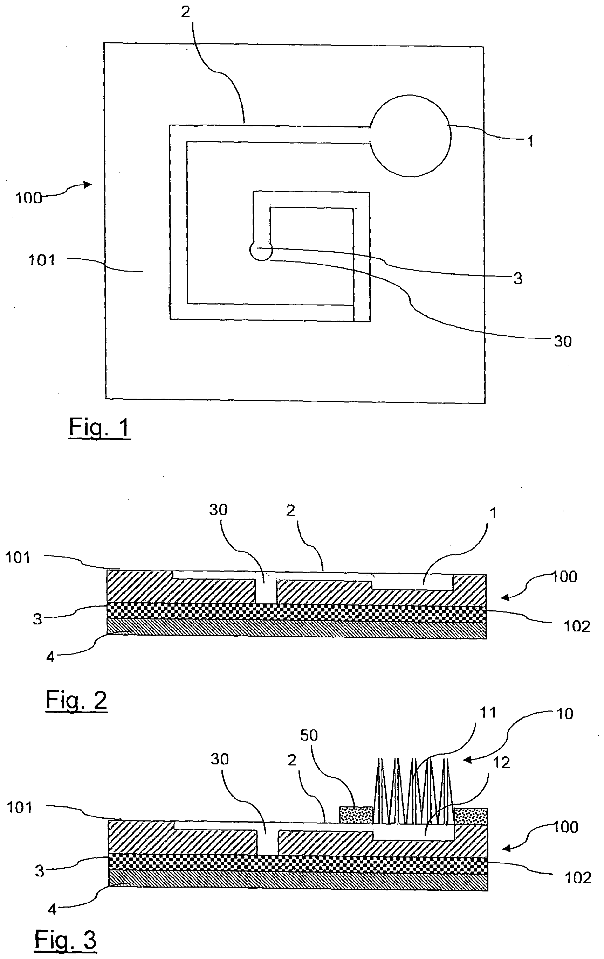

La section d'aliquotage et dispense du sang des figures 1 et 2 présente une structure planaire et est réalisée, par exemple par moulage ou usinage, à partir d'un élément 100 en matière plastique dure ou souple. Un plastique mou tel que du polydimethylsiloxane (PDMS) ou une silicone est particulièrement préféré pour la mise en oeuvre de l'invention de même qu'un plastique moulable ou usinable transparent et de préférence hydrophile.

Sur une surface dite supérieure 101 de l'élément 100 sont formés : un puits ou cuvette 1, destiné à la collecte du sang entier à aliquoter et filtrer, et un conduit capillaire 2 fermé, en communication fluidique avec le puits 1. Un capot doit être disposé au-dessus du conduit 2.

A son extrémité éloignée du puits 1, le conduit 2 débauche dans un trou 30 qui traverse l'élément 100. Sur la surface dite inférieure 102 de cet élément, se trouve une membrane filtrante 3, typiquement en papier.

Au-dessous de cette membrane peut se trouver un élément absorbant 4, dont la fonction sera expliquée plus loin.

Le volume interne de l'élément de collecte 1 et du conduit capillaire déterminent, ensemble, la quantité de sang qui peut être dispensée. Ce volume est généralement compris entre 10 µl et 1 ml, et de préférence entre 50 µl et 500 µl. Il est préférable, pour optimiser la précision de l'aliquotage, que le volume du conduit 2 soit largement supérieur par rapport à celui du puits 1 : pour cette raison ledit conduit peut être enroulé en spirale ou agencé en serpentin. Globalement, le dispositif a des dimensions de l'ordre des centimètres. Plus précisément, le puits de collecte 1 peut avoir un diamètre de l'ordre de 1 - 10 mm, et le conduit capillaire présenter une section de quelques mm² d'aire de plusieurs centimètres de longueurs ; par exemple on peut envisager un conduit de section rectangulaire 0,5 x 2 mm, avec une longueur de 7,5 cm, soit un volume interne de 75 µl.

Le conduit capillaire 2 et/ou le puits 1 peuvent contenir, avant utilisation, divers composés liquides, en gel ou séchés tels que des agents anticoagulants pour le sang tel que l'acide éthylène-diamine-tétra-acétique (EDTA), des agents agglutinants de globules tels que la lectine, ou des agents provocant la coagulation tels que la prothrombine. Il est particulièrement avantageux que les parois internes du conduit 2 soient induites d'un gel qui rend lesdites parois hydrophiles et qui relâche progressivement un anticoagulant lors du remplissage du capillaire, pour empêcher ainsi tout début de coagulation.

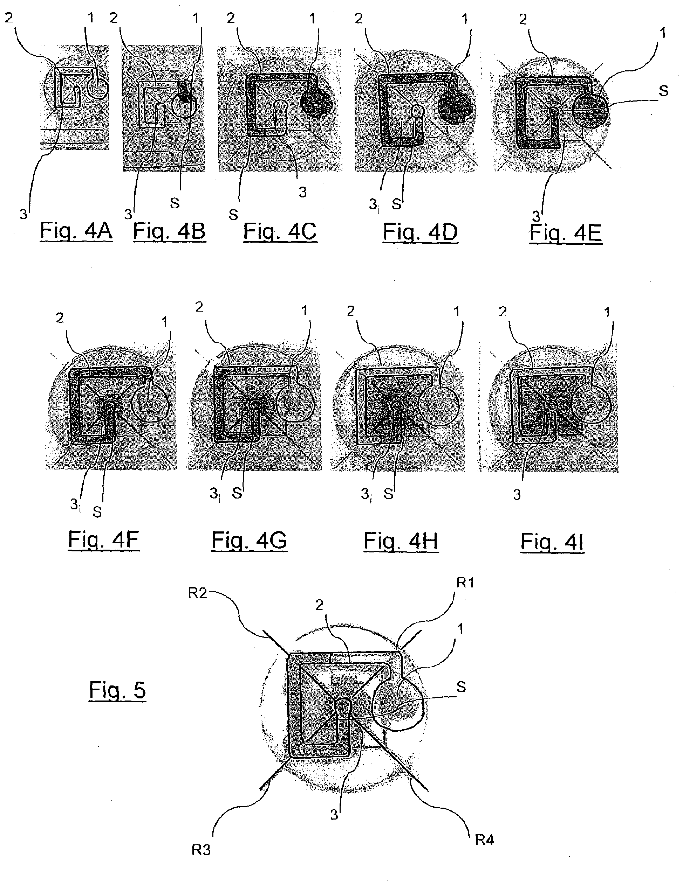

Comme le montrent les figures 4A à 4E, des gouttelettes d'un sang S peuvent être déposées une par une dans le puits 1, le remplissant progressivement. Quand le puits 1 est suffisamment rempli, le sang S commence à pénétrer dans le conduit 2, et y est aspiré par capillarité. Lorsque le conduit 2 est complètement rempli, le sang S atteint la membrane filtrante 3 par l'intermédiaire du trou 30, et est aspiré par cette dernière, qui présente une force de capillarité sensiblement plus grande de celle du conduit 2. A ce point, on arrête de déposer des gouttelettes dans le puits 1. Comme le volume du puits 1 et du conduit 2 sont calibrés, la quantité de liquide à l'intérieur du dispositif est connue de manière précise : il y a donc aliquotage.

Dans certains cas, ne faisant pas partie de l'invention, l'aspiration du sang S par la membrane filtrante 3 se poursuit, jusqu'au vidage complet du dispositif (figures 4F à 4I): il y a donc dispense de la quantité aliquotée de sang.

La membrane filtrante 3 est adaptée pour retenir les cellules sanguines et laisser passer le plasma. Ce dernier peut donc être collecté en aval de ladite membrane.

En particulier, le plasma sanguin peut être collecté par un élément absorbant 4 en contact avec la membrane filtrante 3 et détachable du dispositif. L'élément absorbant 4, s'imbibe de plasma, puis il est séparé et séché. A ce point, il peut être envoyé à un laboratoire pour l'analyse biochimique des protéines qui y restent stockées : cette application est décrite en détail dans la demande de brevet en France FR 07 07709 déposée le 2 novembre 2007.

Selon l'invention, la membrane filtrante 3 peut être dimensionnée de manière à se saturer en globules sanguins avant le vidage complet du dispositif. La quantité exacte de sang qui peut traverser le filtre avant que tous les pores de ce dernier ne soient obstrués par les cellules sanguines (globules rouges, blancs, plaquettes) dépend de son taux d'hématocrite. Ce taux peut donc être estimé en mesurant la quantité du liquide qui reste à l'intérieur du dispositif après le vidage partiel de ce dernier. Cette mesure peut être réalisée de manière approximative à l'aide de repères visuels (traits R1 - R4 sur la figure 5) dont est pourvu le dispositif. On comprend que dans ce cas l'élément planaire 100 doit être transparent.

Selon encore un autre mode de réalisation, le plasma peut être récolté dans un bac disposé en aval du filtre 3.

Un réseau de micropiliers peut également constituer un élément de dispense adapté à la mise en oeuvre de l'invention. Par exemple, il peut s'agir de micropiliers en silicium, ayant une section carrée de 50 µm x 50 µm, une séparation également de 50 µm et une hauteur de 200 µm. Ces micropiliers peuvent être induits de substances chimiques adaptées (par exemple liaison anticorps-antigènes, streptavidin) pour réaliser un prétraitement du sang comme par exemple capter des analytes (Arn, protéines) qui pourront ensuite être détectées à travers le dispositif ou collectée pour analyse.

La figure 3 montre une variante particulièrement avantageuse du dispositif de l'invention, comportant un élément de collecte 10 constitué par une matrice de microaiguilles 11, de préférence creuses, et par un réservoir 12 intégré à l'élément planaire 100 et disposé au-dessous de ladite matrice.

Les microaiguilles 11 sont adaptées pour traverser la peau d'un patient, par exemple au niveau de l'extrémité d'un doigt, et extraire une faible quantité de sang ; elles doivent donc avoir une longueur d'au moins 200 µm, et de préférence de 1 à 2 mm, pour pénétrer dans le derme. De cette manière on minimise le risque d'infection pour le patient et de contamination de l'échantillon prélevé. De plus, l'extraction du sang, son aliquotage et sa dispense sont effectués par une opération unique.

De préférence, une région de collage 50, par exemple en forme d'anneau, entoure l'élément de collecte. Dans le cas d'un prélèvement sanguin, le patient appuie son doigt au niveau dudit élément de collecte, le cas échéant après s'être piqué avec une lancette (si le dispositif ne comporte pas de microaiguilles). La région collante 50 permet la formation d'un joint étanche entre le doigt et le dispositif, évitant toute dispersion de sang et les risques biologiques connexes ainsi qu'un contact prolongé entre le sang et l'air, susceptible d'accélérer la coagulation). Le patient garde son doigt sur le dispositif pendant toute la phase de remplissage. Lorsque le conduit 2 est plein, il enlève le doit, ce qui permet un vidage rapide du dispositif par capillarité : en effet, tant que le doigt est présent, le vidage est ralenti et limité au débit d'écoulement du sang, alors que lorsque le doigt est enlevé le débit est contrôlé par la force de capillarité en sortie du dispositif. Le joint collant n'empêche pas le massage du doigt qui aide à l'extraction du sang. L'élément de collecte peut également comporter de piliers plus ou moins souples qui aident à masser localement le doigt facilitant ainsi l'écoulement du sang du doigt vers le dispositif.

## Revendications

1. Dispositif d'aliquotage, de filtrage et de mesure du taux d'hématocrite du sang d'un patient, comportant :
- un élément (1, 10) de collecte de sang (S) ;
- un conduit capillaire (2), présentant une force de capillarité supérieure à celle dudit élément de collecte et en communication fluidique avec lui ; et
- un élément de dispense (3), présentant une force de capillarité supérieure à celle du conduit capillaire et en communication fluidique avec lui ; ledit élément de dispense comportant un dispositif de filtrage pour séparer des globules sanguins du plasma ;
le dispositif étant **caractérisé en ce que** ledit dispositif de filtrage est adapté pour se saturer en globules après le filtrage d'un volume de sang dépendant du taux d'hématocrite du dit sang mais, en tout cas, inférieur à la contenance dudit élément de collecte et dudit conduit capillaire ; et dans lequel des repères visuels (R1 - R4) sont prévus, permettant d'estimer par simple inspection le volume de liquide présent dans ledit conduit capillaire ; moyennant quoi le taux d'hématocrite dudit sang peut être déterminé à partir de l'estimation du volume de sang restant dans le conduit capillaire après saturation du dispositif de filtrage.

2. Dispositif selon la revendication 1, dans lequel ledit élément de collecte (1) est un puits ou cuvette.

3. Dispositif selon la revendication 1, dans lequel ledit élément de collecte comporte une matrice de microaiguilles (11) et un réservoir (12) disposé au-dessous de cette dernière.

4. Dispositif selon l'une des revendications précédentes, dans lequel une zone de collage (50) est prévue sur une surface (101) dudit dispositif, en correspondance dudit élément de collecte (10), de manière à permettre un contact étanche entre ledit élément de collecte et une zone de la peau d'un patient.

5. Dispositif selon l'une des revendications précédentes, dans lequel ledit élément de collecte et ledit conduit capillaire sont adaptés pour contenir un volume de sang compris entre 10 µl et 1 ml, et de préférence entre 50 µl et 500 µl.

6. Dispositif selon l'une des revendications précédentes, dans lequel ledit élément de collecte et/ou ledit conduit capillaire contiennent au moins une substance chimiques choisie parmi :
- des agents anticoagulants pour le sang ;
- des agents agglutinants des globules sanguins ; et
- des agents coagulants pour le sang.

7. Dispositif selon la revendication 6, dans lequel au moins les parois internes dudit conduit capillaire sont induites d'un gel hydrophile qui relâche progressivement un agent anticoagulant du sang lors de son remplissage par du sang à aliquoter et filtrer.

8. Dispositif selon l'une des revendications précédentes, dans lequel ledit élément de collecte et ledit conduit capillaire sont intégrés dans un même élément de structure planaire (100).

9. Dispositif selon la revendication 8, dans lequel ledit élément de structure planaire est transparent.

10. Dispositif selon l'une des revendications 8 ou 9, dans lequel ledit élément de structure planaire est réalisé par moulage ou usinage dans un plastique.

11. Dispositif selon l'une des revendications précédentes, dans lequel ledit élément de collecte (1, 10) et ledit conduit capillaire (2) sont intégrés sur une surface (101) d'un même élément de structure planaire (100), ledit dispositif de filtrage (3) comportant une membrane disposée sur une face opposée (102) dudit élément, le conduit capillaire et le dispositif de filtrage étant mis en communication fluidique par un trou (30) traversant cet élément.

12. Dispositif selon l'une des revendications précédentes, dans lequel ledit dispositif de filtrage (3) est un filtre en papier.

13. Dispositif selon l'une des revendications 11 et 12, adapté pour effectuer des prélèvements sanguins et comportant également, en aval dudit dispositif de filtrage (3), un élément détachable (4) en matériau absorbant le plasma sanguin et apte à conserver, après séchage, les protéines contenues dans ledit plasma.

14. Procédé de mesure du taux d'hématocrite du sang au moyen d'un dispositif selon l'une des revendications précédentes, comportant les opérations suivantes :
(a) déposer du sang (S) dans l'élément de collecte (1, 10) dudit dispositif jusqu'à ce que le sang soit aspiré par le dispositif de filtrage (3) ;
(b) arrêter de déposer du sang dans l'élément de collecte (1, 10) ;
(c) attendre que le dispositif de filtrage se sature en globules sanguins ; et
(d) estimer par simple inspection, à l'aide des repères visuels (R1 - R4), le volume de liquide restant dans ledit conduit capillaire, et en déduire une estimation du taux d'hématocrite recherché.

## Patentansprüche

1. Vorrichtung zur Aliquotierung, zur Filterung und zur Messung des Hämatokritwerts von Blut eines Patienten, umfassend:
- ein Element (1, 10) zur Sammlung von Blut (S);
- eine Kapillarleitung (2), die eine Kapillaritätskraft aufweist, die größer als diejenige des Elements zur Sammlung und in fluidischer Kommunikation mit ihm ist; und
- und ein Element zur Abgabe (3), das eine Kapillaritätskraft aufweist, die größer als diejenige der Kapillarleitung und in fluidischer Kommunikation mit ihr ist; wobei das Element zur Abgabe eine Filtervorrichtung umfasst, um die Blutkörperchen vom Plasma zu trennen;
- wobei die Vorrichtung **dadurch gekennzeichnet ist, dass** die Filtervorrichtung ausgelegt ist, um sich nach der Filterung eines Blutvolumens mit Körperchen zu sättigen, je nach dem Hämatokritwert des Bluts, das jedoch in jedem Fall kleiner als der Inhalt des Elements zur Sammlung und der Kapillarleitung ist; und wobei visuelle Anhaltspunkte (R1 - R4) vorgesehen sind, die ermöglichen, durch eine einfache Inspektion das Volumen der Flüssigkeit zu schätzen, die in der Kapillarleitung vorhanden ist; wodurch der Hämatokritwert des Bluts auf der Grundlage der Schätzung des Blutvolumens bestimmt werden kann, das nach der Sättigung der Filtervorrichtung in der Kapillarleitung bleibt.

2. Vorrichtung nach Anspruch 1, wobei das Element zur Sammlung (1) eine Vertiefung oder eine Schüssel ist.

3. Vorrichtung nach Anspruch 1, wobei das Element zur Sammlung eine Matrix aus Mikronadeln (11) und einen Behälter (12) umfasst, der unter dieser Letzteren angeordnet ist.

4. Vorrichtung nach einem der vorhergehenden Ansprüche, wobei ein Bereich zum Aufkleben (50) auf einer Fläche (101) der Vorrichtung vorgesehen ist, in Übereinstimmung mit dem Element zur Sammlung (10), um einen engen Kontakt zwischen dem Element zur Sammlung und einem Bereich der Haut eines Patienten zu ermöglichen.

5. Vorrichtung nach einem der vorhergehenden Ansprüche, wobei das Element zur Sammlung und die Kapillarleitung ausgelegt sind, um ein Blutvolumen zu enthalten, das zwischen 10 µl und 1 ml und vorzugsweise zwischen 50 µl und 500 µl liegt.

6. Vorrichtung nach einem der vorhergehenden Ansprüche, wobei das Element zur Sammlung und/oder die Kapillarleitung mindestens eine chemische Substanz enthalten, ausgewählt aus:
- Antikoagulationsmitteln für das Blut,
- Agglutinationsmitteln der Blutkörperchen; und
- Koagulationsmitteln für das Blut.

7. Vorrichtung nach Anspruch 6, wobei mindestens die Innenwände der Kapillarleitung mit einem hydrophilen Gel beschichtet sind, das progressiv ein Antikoagulationsmittel des Bluts freisetzt, wenn sie mit dem Blut zur Aliquotierung und Filterung gefüllt wird.

8. Vorrichtung nach einem der vorhergehenden Ansprüche, wobei das Element zur Sammlung und die Kapillarleitung in einem gleichen Element mit planarer Struktur (100) integriert sind.

9. Vorrichtung nach Anspruch 8, wobei das Element mit planarer Struktur transparent ist.

10. Vorrichtung nach einem der Ansprüche 8 oder 9, wobei das Element mit planarer Struktur durch Formen oder Bearbeitung in einem Plastik hergestellt ist.

11. Vorrichtung nach einem der vorhergehenden Ansprüche, wobei das Element zur Sammlung (1, 10) und die Kapillarleitung (2) auf einer Fläche (101) eines gleichen Elements mit planarer Struktur (100) integriert sind, wobei die Filtervorrichtung (3) eine Membran umfasst, die auf einer gegenüber liegenden Seite (102) des Elements angeordnet ist, wobei die Kapillarleitung und die Filtervorrichtung durch ein Loch (30), das dieses Element quert, in fluidische Kommunikation versetzt wird.

12. Vorrichtung nach einem der vorhergehenden Ansprüche, wobei die Filtervorrichtung (3) ein Filter aus Papier ist.

13. Vorrichtung nach einem der Ansprüche 11 und 12, die ausgelegt ist, um Blutentnahmen durchzuführen und auch, nachgelagert von der Filtervorrichtung (3), umfassend ein abnehmbares Element (4) aus einem Material, das das Blutplasma absorbiert und ausgelegt ist, um nach dem Trocknen die Proteine zu konservieren, die in dem Plasma enthalten sind.

14. Verfahren zum Messen des Hämatokritwerts des Bluts mit Hilfe einer Vorrichtung nach einem der vorhergehenden Ansprüche, umfassend die folgenden Vorgänge:
(a) Ablegen des Bluts (S) im Element zur Sammlung (1; 10) der Vorrichtung, bis das Blut von der Filtervorrichtung aufgesaugt (3) ist;
(b) Stoppen der Ablage von Blut im Element zur Sammlung (1; 10);
(c) Warten, bis sich die Filtervorrichtung mit Blutkörperchen sättigt; und
(d) Schätzen, durch eine einfache Inspektion, mit Hilfe der visuellen Anhaltspunkte (R1 - R4), des Volumens der Flüssigkeit, die in der Kapillarleitung bleibt, und davon Ableiten einer Schätzung des gesuchten Hämatokritwerts.

## Claims

1. A device for aliquoting and filtering blood, the device comprising:
• a collector element (1, 10) for collecting blood (S) ;
• a capillary duct (2) presenting a capillary force stronger than that of said collector element and in fluid flow communication therewith; and
• a dispenser element (3) presenting a capillary force stronger than that of the capillary duct and in fluid communication therewith; said dispenser element including a filter device (3) for separating blood corpuscles from plasma; the device being **characterized in that** said filter device is adapted to become saturated in corpuscles after filtering a volume of blood that depends on the hematocrit content of said blood and that is in any event less than the content of said collector element plus said capillary duct; and wherein visible marks (R1 - R4) are provided that make it possible, by mere inspection, to estimate the volume of liquid present in said capillary duct; thereby enabling the hematocrit content of said blood to be determined on the basis of an estimate of the volume of blood remaining in the capillary duct after the filter device has saturated

2. A device according to claim 1, wherein said collector element (1) is a well or depression.

3. A device according to claim 1, wherein said collector element includes an array of microneedles (11) and a reservoir (12) located beneath the array.

4. A device according to any preceding claim, wherein an adhesive zone (50) is provided on a surface (101) of said device in register with said collector element (10) so as to provide leaktight contact between said collector element and a zone of a patient's skin.

5. A device according to any preceding claim, wherein said collector element and said capillary duct are adapted to contain a volume of blood lying in the range 10 µL to 1 mL, and preferably in the range 50 µL to 500 µL.

6. A device according to any preceding claim, wherein said collector element and/or said capillary duct contain at least one chemical substance selected from:
• anticoagulating agents for blood;
• agglutinating agents for blood corpuscles; and
• coagulating agents for blood.

7. A device according to claim 6, wherein at least one of the inside walls of said capillary duct is coated in a hydrophilic gel that progressively releases a blood anticoagulating agent while the duct is being filled with blood for aliquoting and filtering.

8. A device according to any preceding claim, wherein said collector element and said capillary duct are integrated in a common element of plane structure (100).

9. A device according to claim 8, wherein said element of plane structure is transparent.

10. A device according to claim 8 or claim 9, wherein said element of plane structure is made by molding or machining a plastics material.

11. A device according to any preceding claim, wherein said collector element (1, 10) and said capillary duct (2) are incorporated in a surface (101) of a single element (100) of plane structure, said filter device (3) comprising a membrane placed on an opposite face (102) of said element, the capillary duct and the filter device being put into fluid flow communication via a hole (30) passing through the element.

12. A device according to any preceding claim, wherein said filter device (3) is a paper filter.

13. A device according to any one of claims 11 and 12, adapted to take blood samples and also including, downstream from said filter device (3), a detachable element (4) of material that absorbs blood plasma and that, on being dried, is suitable for conserving the proteins contained in said plasma.

14. A process for measuring the hematocrit content of blood by means of a device according to any one of the preceding claims, comprising the following steps:
(a) deposit blood in the collector element (1,10) of said device until blood is sucked up by the filter device (3);
(b) stop depositing blood in the collector element (1; 10) ;
(c)wait for filter device to saturate in blood corpuscules; and
(d) estimate by mere inspection, thanks to visible marks (R1-R4), the volume of liquid present in said capillary duct, and deduce an estimate of the hematocrit content.
